# EUROPEAN PATENT SPECIFICATION

(11) **EP 1 873 292 B1**
(45) Date of publication and mention of the grant of the patent: **11.11.2009**
(21) Application number: 06013106.7
(22) Date of filing: 26.06.2006
(51) Int. Cl.: D04H 13/00, A61F 13/15

(54) **Multicomposite variable-density multilayer non-woven fabric and method for manufacturing it**
Mehrschichtiges und multikomponentes Vliesmaterial mit unterschiedlicher Faserdichte und Verfahren zu dessen Herstellung
Tissu non-tissé multicouche et multi-composant présentant des zones de densité différentes ainsi que sa méthode de fabrication

(43) Date of publication of application: 02.01.2008
(73) Proprietor: Maranghi Marco, 59100 Prato (IT)
(72) Inventor: Maranghi Marco, 59100 Prato (IT)
(74) Representative: Forattini, Amelia

(56) References cited:
- EP-A- 1 359 241
- EP-A2- 0 613 671
- WO-A-01/54641
- WO-A-97/18783
- WO-A-2005/012620
- WO-A2-2005/044177
- US-A- 4 027 672
- US-A- 5 451 442

## Description

The present invention relates to a multicomposite variable-density multilayer non-woven fabric and to the method for manufacturing it.

The non-woven fabric according to the present invention can be used advantageously in the field of sanitary towels and diapers.

According to the prior art, sanitary towels for women and diapers in general, both for children and for incontinent adults, are manufactured by joining and assembling and fusing together various materials in suitable machinery.

As many as five different components, or even more, are normally used, with two external containment films inside which there are various internal layers with different functions. By way of example, according to the prior art the towels or diapers are constituted by a top sheet, which consists of a first outer film, which can be formed by a perforated polyethylene film or by a non-woven fabric or others, is capable of providing the so-called dry effect on the skin and allows the passage of the biological liquid to the underlying layers. Internally, according to the prior art, there is a liquid storage region, which consists of a second layer designed to acquire and distribute the liquid (acquisition layer), a third absorbent (air-laid) layer, mostly formed by a sheet arranged in a closed C-shape, suitable to contain a fourth component, constituted by superabsorbent powders. Below this liquid storage region there is a fifth component, which consists of a lower protective and impermeable film, usually made of polyethylene, which prevents the outflow of the absorbed liquids.

According to the prior art, machines for producing sanitary towels for female hygiene and diapers in general must assemble the various components cited above, taking care to insert correctly the superabsorbent powders inside the third layer.

This known production has many drawbacks, the main one being, from an industrial point of view, that the machines for manufacturing sanitary towels and/or diapers are extremely expensive, because they must assemble the various components, that are usually fused along their edges.

The drawback of conventional sanitary towels is the considerable volume and weight that must be achieved in order to obtain a certain degree of absorption. Although sanitary towels have a certain length, the absorbed liquid in fact is not diffused along the entire towel, but remains concentrated only in the gathering point, at best spreading in a stain-like fashion, leaving all the rest of the sanitary towel unused along its length. When the liquid reaches a certain quantity, due to the inability to provide absorption which is distributed correctly over the length of the product, the liquid may also exit from the lateral edges. This drawback is particularly incovenient in the case of sanitary towels for women.

All of the drawbacks cited above have been overcome by the multicomposite variable-density multilayer non-woven fabric according to the present invention.

The aim of the present invention is to provide a composite material which directly contains a differentiated structure having the necessary characteristics, which constitutes a semifinished product, in a continuous sheet, enormously simplifying the machines for manufacturing sanitary towels and/or diapers, at the same time reducing the weight and thickness of the finished item.

The multilayer non-woven fabric according to the invention has been provided for this purpose and, by being multicomposite with a preset variable density, considerably increases the efficiency of the product.

In order to achieve this aim and to make a better use of the surface of the product, the fabric has been provided with longitudinal channels, increasing the speed and consequently the capacity to distribute the liquid on the surface of the product.

Some of the numerous advantages obtained with the present invention are as follows.

The main advantage is that by increasing the liquid absorption surface it has accordingly been possible to decrease the thickness of the product for achieving the required degree of absorption while maintaining the necessary dry effect and at the same time improving manufacturing economy.

Another advantage is that the efficiency, softness and pleasantness of the product are increased, those features being particularly important when the product is used for manufacturing sanitary towels for women, so-called panty liners, or diapers for children.

Another advantage is that a considerable overall saving is achieved in the machines for manufacturing the finished product, because the machines no longer have to assemble the many different components but have a single continuous sheet to be processed by die-cutting it according to the requirements.

In order to manufacture the multilayer non-woven fabric according to the invention, a carded ply was produced with a material which has absorbent characteristics, such as viscose or cotton or cellulose fibers, or a material known by the trade name Lyocell, or again with a mixture of these fibers with polyester or polyolefin fibers in general, such as polyester (PET), polypropylene (PP), polylactic acid (PLA). The material was bonded with a spunlacing system, producing a bonded ply.

In any case, it is possible to use as a bonded ply a non-woven fabric (air-laid fabric) produced with known methods and formed by cellulose fibers or mixtures of cellulose and/or heat-binding fibers.

A non-bonded ply, made of a material with fibers which are not absorbent but are in any case hydrophilic, is deposited on the bonded ply. The non-bonded ply is joined to the bonded ply with a spunlacing system, that binds the two films only along parallel lines. This produces, in the non-bonded ply, a reduction in volume along the binding lines, which form recesses, while longitudinal pads remain in the gaps between the binding lines. The fibers of the non-bonded ply in the gaps remain soft and free. A composite product is thus provided.

The non-bonded ply is preferably provided beforehand so that the fibers have a randomized orientation, in order to allow the fibers to arrange themselves also transversely, with respect to the binding lines, so as to prevent their extraction and to prevent loss of consistency of the product.

The non-bonded ply can be formed by fibers chosen among: polyesters (PET), polypropylene (PP), polyethylene (PE), polyamide (PA), polylactic acid (PLA) or polyesters or polyolefins in general or mixtures thereof.

If liquid is poured onto the composite product, it passes from the upper ply to the underlying ply, and after a few seconds one obtains the dry effect, due to the hydrophilic nature of the fibers of the upper ply and to the absorption of the fibers of the lower bonded ply.

Because of the binding lines provided between the two plies, the wetness on the lower ply extends longitudinally, parallel to the binding lines, and is more diffused than in conventional towels.

The binding lines, where the density of the non-bonded ply becomes greater, constitute barriers to the diffusion of the liquid in a transverse direction, guiding the liquid along the gaps between the lines, where the density of the material in the non-bonded ply has remained minimal, allowing the liquid to spread rapidly along these longitudinal pads as if there were channels.

The longitudinal pads in which the fibers remain soft and free and have a lower density remain on the upper surface designed to make contact with the user, and this softness gives the product a pleasant, comfortable and welcomed smoothness. Another improvement due to the new structure is the substantial reduction of the so-called "wet back" (rewetting) effect, which consists of the rise of the fluid to the surface in contact with the user if pressed. Such reduction is due to the greater diffusion of the fluid on the entire surface of the article and of the consequent reduction of the concentration of fluid in the central parts thereof.

The composite product can be processed further by depositing on the surface of the lower part of the bonded ply a mixture of powders of polyethylene, polyvinyl acetate (EVA), polypropylene (PP), or polyolefin powders or preferably powders of polylactic acid, mixed with superabsorbent powders.

The composite product, with a thin layer of powders on the rear surface, is made to pass through a field of infrared rays, which melt the polyethylene, polylactic acid, polyvinyl acetate, polypropylene or polyolefin powders, and bind the superabsorbent powders to the rear surface of the composite product, forming a thin film which distributes and bonds the superabsorbent powders instead of inserting them, according to the prior art, inside the C-shaped sheet of the third layer. This operation is evidently far simpler and less expensive than the prior art operations and also allows to reduce the loss of superabsorbent powders caused by subsequent processes and shaking of the fabric during processing. The non-woven fabric thus composed can be already coupled, in this step or in a subsequent step, to a barrier film, which in turn can be composed of one or more laminated layers, the internal one being made of non-woven fabric made of synthetic fibers, cellulose fibers or mixtures thereof.

According to another example of application of superabsorbent powders, a non-woven fabric is laminated or coupled to a film of polyethylene or polylactic acid or thermoplastic polymers, the non-woven fabric being preferably made of viscose fibers or cotton fibers (in order to obtain a product which is 100% biodegradable) or of a mixture of fibers with thermoplastic fibers such as polyester, polypropylene or polymeric or polyolefin fibers or polylactic acid fibers. This non-woven fabric is applied to the surface layer of adhesive by hot melting with known methods, such as spreading, spraying or transfer with a hot roller, with the graining roller technique, and then the superabsorbent powder is immediately distributed uniformly and arranged in longitudinal lines a few millimeters wide, with interleaved empty spaces, by using systems which are commonly known and are described, for example, in US-6511704, US-5895542, and US-4264644. After the distribution of the superabsorbent powder, which is fixed to the melted polymers by melting of the resins (hot melt), the fabric described above is coupled and is fixed in the still-empty spaces left by the absence of the superabsorbent powder, and couples so as to leave the superabsorbent powder inside the two layers.

A multicomposite non-woven fabric is thus obtained.

The addition of the powders gives the multicomposite non-woven fabric a greater absorption capacity, but the correct diffusion of the liquid also allows a considerable saving in the amount of absorbent powders to be used.

The film formed by the superabsorbent powders can be approximately 5 to 15 micrometers thick.

All the advantages described above and others will become better apparent from the description of the figures that follow
Figure 1 is a schematic side section view of the bonded ply;
Figure 2 is a side section view of the non-bonded ply placed over the bonded ply;
Figure 3 is a side section view of the composite product;
Figure 4 is a perspective view of the non-woven fabric according to the invention;
Figure 5 is a schematic view of the manufacturing method of the variable-density composite product;
Figure 6 is a schematic view of the method of another treatment which relates to the deposition of the superabsorbent powders.

In the various figures, similar elements have been designated by the same reference numerals.

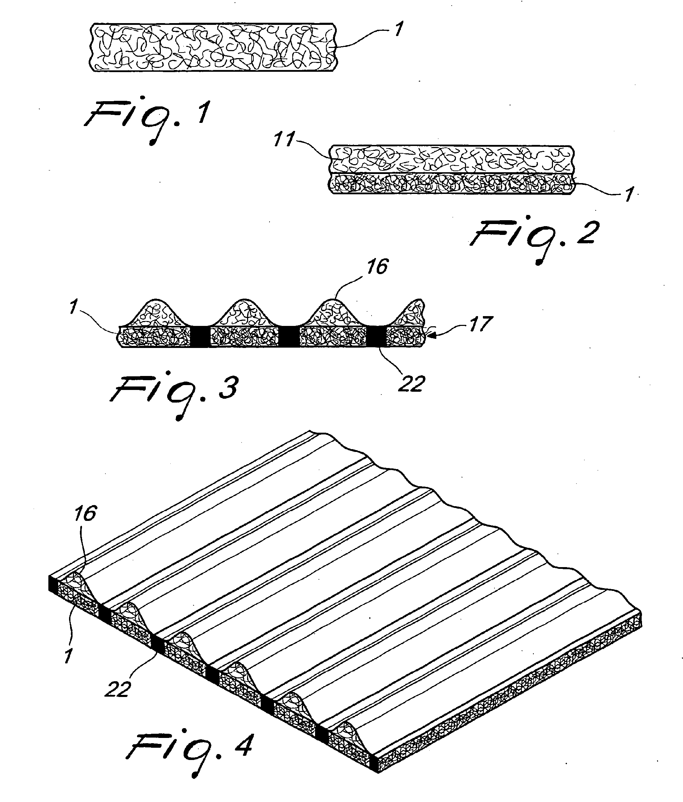

Figure 1 is a schematic view of a bonded ply 1.
Figure 2 is a view of a non-bonded ply 11 rested on the bonded ply 1.
Figure 3 is a section view of a composite product 17, constituted by the non-bonded ply 11 rested on the bonded ply 1, after producing bindings along parallel lines. Along the bindings, in the points 22, troughs are formed in the ply 11, increasing its density and constituting barriers. In the ridges 16, the ply 11 instead maintains its looseness, forming longitudinal pads which constitute channels for distributing the liquid to be absorbed.
Figure 4 is a view of a non-woven fabric provided according to the present invention, in which the ridges 16 and the bindings 22 are visible.

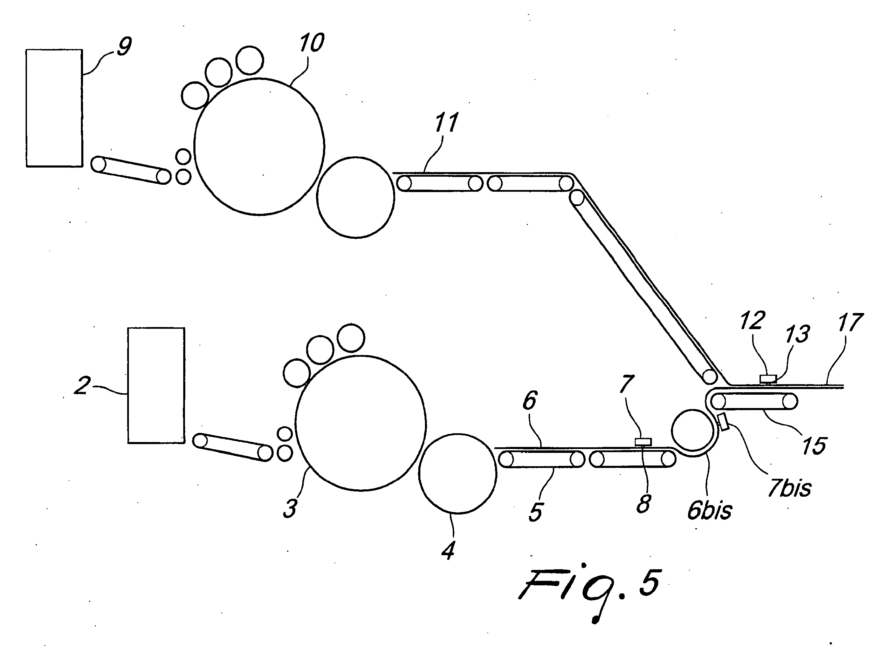

As visible in Figure 5, the fibers are fed from a magazine 2 onto a carding system 3, 4, which opens the fibers, that are arranged transversely or randomized, and thus form a ply of fibers 6 rested on a conveyor 5. The ply of fibers 6 is bonded on one face in a station 7 by means of high-pressure water jets. The water jets entangle the fibers with the operation commonly known as spunlacing, passing through microperforated strips 8, known as jet strips, which consist of a plate with an extremely large number of mutually adjacent small holes which are mutually spaced by approximately 0.8-1 mm. The ply 6bis, bonded on one face, is sent to a second station 7bis, which bonds it on the other face, forming the bonded ply 1, which is rested on a conveyor 15.

With a similar method, a magazine 9 provides the fibers to a carding machine 10, which forms a non bonded ply of fibers 11. The ply 11 is deposited over the bonded ply 1, on the conveyor belt 15. The two superimposed plies 1-11 pass through a station 12 of high-pressure water jets. In the station 12, the finely perforated strip 13, the jet strip, has its holes arranged along parallel lines which are spaced by 2 to 15 mm, providing the composite product 17, with the binding components 22 spaced consequently and the corresponding ridges 16. Finally, the composite product 17 is usually rolled onto a reel.

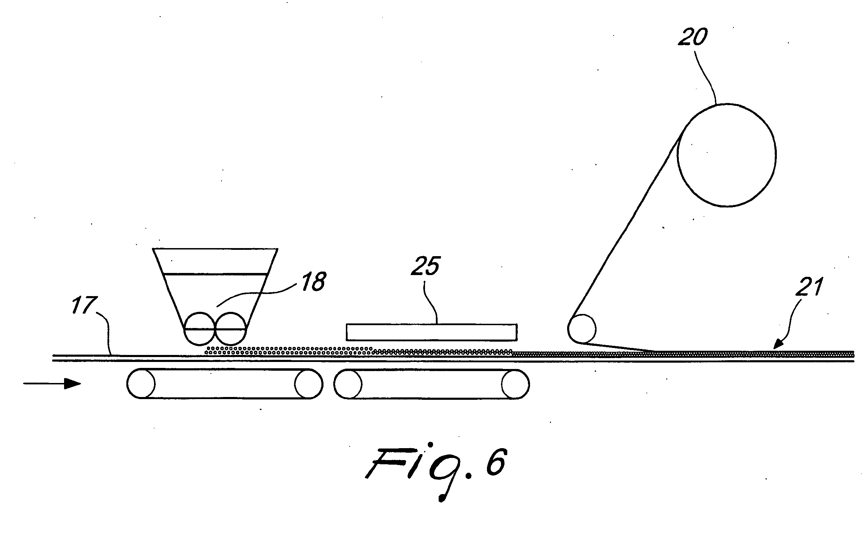

Figure 6 is a diagram related to the additional treatment regarding the deposition of the superabsorbent powders onto the composite product 17.

The composite product 17 is provided by the reel in an inverted position, i.e., so that the lower face of the product is arranged above and rested on a conveyor belt 24. The conveyor 24 passes the product 17 below a powder feeder 18, which deposits the powders onto the back of the surface of the product 17. The product 17 then passes through a field of infrared rays, which melt the polyethylene, polylactic acid, polyvinyl acetate, polypropylene or the polyolefin powders mixed with the superabsorbent powders, and bind the superabsorbent powders to the rear surface of the composite product 17, forming a thin film which distributes the superabsorbent powders.

In order to prevent the unwanted escape of the absorbed liquids, it is possible to provide an additional roll 20, which provides a film of polyethylene or polylactic acid, with a thickness of 10 to 25 micrometers, which is coupled directly to the rear surface of the product 17, while the polyethylene or polylactic acid, already deposited with the superabsorbent powders, are still melted.

The reference numeral 21 designates the final product, which is constituted by a multicomposite variable-density multilayer non-woven fabric.

The above described materials may be matched in different ways: in particular, if one wishes to obtain a product which is 100% biodegradable, one uses fibers of polylactic acid for the non-bonded ply, otherwise it is possible to use cheaper polypropylene or polyester. Accordingly, for the superabsorbent powders, one can use the ones known by the trade name LYSAC, that are organic and 100% biodegradable, or the ones commonly known as SAP.

According to a preferred embodiment, a mixture of 20% polyethylene or polylactic acid powder with 80% superabsorbent powder is used.

According to a preferred embodiment, the bonded ply is constituted by a layer of 65 g/m2 with a 3.2-dtex viscose fiber.

The binding lines are provided at a distance between 2 and 15 mm.

According to the description of the cited examples, a reduction in the thickness of the sanitary towel from the conventional 3 mm down to 1.2-1.5 mm has been achieved, allowing greater smoothness and better diffusion of the liquid.

## Claims

1. A non-woven fabric, which is a multilayer variable-density fabric with longitudinal channels, comprises a non-bonded ply (11), having non-absorbent hydrophilic characteristics which rests on a bonded ply (1), having absorbent characteristics, said plies being bound to each other along parallel lines (22), **characterized in that** said bonded ply (1) is constituted by a carded ply (6) bonded with a spunlacing system, said non-bonded ply (11) being connected to said bonded ply (1), along said binding lines (22), and having troughs and longitudinal ridges (16); said longitudinal ridges (16) extending along gaps between said binding lines (22); said bonded ply (1) having a rear surface, opposite to said non-bonded ply (11), comprising a layer of superabsorbent powders.

2. The non-woven fabric according to claim 1, **characterized in that** said bonded ply (1) is constituted by fibers of viscose or cotton or cellulose, or by a mixture of these fibers with polyester or polyolefin fibers in general, such as polyester (PET), polypropylene (PP), polylactic acid (PLA) and/or heat binding fibers.

3. The non-woven fabric according to claim 1, **characterized in that** the non-bonded ply (11) is made of polylactic acid (PLA) or polyester (PET) or polypropylene (PP) or polyethylene or polyamide fibers or with polyester or polyolefin fibers.

4. The non-woven fabric according to claim 1, **characterized in that** it comprises a a film of polyethylene or polylactic acid, coupled to said surface of said bonded ply (1) opposite to said non-bonded ply (11), over said superabsorbent powders.

5. The non-woven fabric according to claim 1, **characterized in that** said superabsorbent powders are mixed at 80% with 20% of powder of polyethylene or polylactic acid.

6. The non-woven fabric according to claim 1, **characterized in that** said superabsorbent powders are powders commonly known as SAP.

7. The non-woven fabric according to claim 1, **characterized in that** said bonded ply (1) is constituted by a 3.2-dtex viscose fiber with a weight of 65 g/m2.

8. The non-woven fabric according to claim 1, **characterized in that** the binding lines (22) are provided at a distance of 2 to 15 mm.

9. The non-woven fabric according to claim 1, **characterized in that** said layer of superabsorbent powders has a thickness of approximately 5 to 15 micrometers.

10. The non-woven fabric according to claim 4, **characterized in that** said film of polyethylene or polylactic acid has a thickness of 10 to 25 micrometers.

11. A method for manufacturing a non-woven multilayer variable-density fabric with longitudinal channels, **characterized in that** it comprises the steps of:
- providing a carded ply (6) constituted by a material having absorbent characteristics and fibers having a randomized orientation and bonded with a spunlacing system;
- providing a bonded ply (1) made of a material with non-absorbent but in any case hydrophilic fibers;
- depositing said non-bonded ply (11) on said bonded ply (1) and connecting said non-bonded ply (11) to said bonded ply (1) by spunlacing for binding said plies only along binding lines (22), forming thereat troughs in said non-bonded ply (11), while longitudinal ridges (16) remain in the gaps between said binding lines (22), the fibers of the non-bonded ply remaining soft and loose therein, providing a composite product (17);
- depositing a layer of a mixture of powders of polyethylene, polylactic acid, polyvinyl acetate, polypropylene or of polyolefin powders, mixed with superabsorbent powders, on a rear surface of said bonded ply (1) of said composite product (17);
- passing said non-woven fabric through a field of infrared rays, melting said polyethylene, polylactic acid, polyvinyl acetate, polypropylene or polyolefin powders and binding said superabsorbent powders to said rear surface, forming a film.

12. The method according to claim 11, **characterized in that** said carded ply (6) is produced with fibers fed from a magazine (2) onto a carding system (3, 4), which opens the fibers, which are arranged transversely or randomly; said ply of fibers (6) being rested on a conveyor (5) and bonded on one face in a station (7) by means of jets of high-pressure water, forming a bonded ply (6bis) on one face; said ply being sent to a second station (7bis), which bonds it on the other face, forming the bonded ply (1), which is rested on a conveyor (15).

13. The method according to claims 11 and 12, **characterized in that** a magazine (9) provides fibers to a carding machine (10), which forms the non-bonded ply of fibers (11), which is deposited over the bonded ply (1) on the conveyor belt (15); said superimposed plies (1-11) passing through a station (12) of high-pressure water jets, wherein a finely perforated strip (13) has small holes arranged along parallel lines which are spaced by approximately 2 to 15 mm.

14. The method according to claims 11 to 13, **characterized in that** said composite product (17) is provided from a reel (23) upside down, i.e., so that the rear face of the product (17) is arranged above and is rested on a conveyor belt (24), which makes the product (17) pass under a powder feeder (18), which deposits said powders onto said rear surface of the product (17).

15. The method according to claims 11 to 14, **characterized in that** said superabsorbent powders are applied through a non-woven fabric which is laminated or coupled to a film of polyethylene or polylactic acid or other thermoplastic polymers, said non-woven fabric being made of viscose or cotton fibers, or being a mixture of fibers with thermoplastic fibers such as polyester, polypropylene or other polymeric or polyolefin fibers, or polylactic acid fibers; said non-woven fabric being applied to the surface layer of adhesive by hot melting through spreading, spraying or transfer with a hot roller, with a graining roller technique; said superabsorbent powders being distributed uniformly and arranged in longitudinal lines having a width of a few millimeters, with interleaved empty spaces, said fabric being coupled and fixed in the empty spaces left by the absence of the superabsorbent powders, leaving the superabsorbent powders inside the two layers.

16. The method according to claims 11 to 15, **characterized in that** an additional reel (20) provides a film of polyethylene or polylactic acid, possibly already coupled or laminated beforehand with a ply of paper, or with non-woven fabrics, which is coupled directly to the rear surface of the product (17), while the polyethylene or polylactic acid, already deposited with the superabsorbent powders, are still melted.

## Patentansprüche

1. Mehrschichtiger Vliesstoff variabler Dichte mit länglichen Kanälen, umfassend eine auf einer verfestigten Lage (1) mit absorbierenden Eigenschaften ruhende nicht verfestigte Lage (11) mit nicht absorbierenden hydrophilen Eigenschaften, welche entlang paralleler Linien (22) aneinander gebunden sind, **dadurch gekennzeichnet, dass** die verfestigte Lage (1) aus einer mit einem Spunlace-System verfestigten kardierten Lage (6) gebildet ist, wobei die nicht verfestigte Lage (11) mit der verfestigten Lage (1) entlang der Bindelinien (22) verbunden ist und Mulden und längliche Rippen (16) aufweist, wobei die länglichen Rippen (16) entlang von Lücken zwischen den Bindelinien (22) verlaufen, wobei die verfestigte Lage (1) eine der nicht verfestigten Lage (11) gegenüberliegende Rückseite aufweist, die eine Schicht von superabsorbierenden Pulvern umfasst.

2. Vliesstoff nach Anspruch 1, **dadurch gekennzeichnet, dass** die verfestigte Lage (1) aus Viskose- oder Baumwoll- oder Cellulose-Fasern oder einer Mischung dieser Fasern mit Polyester- oder Polyolefinfasern allgemein, wie Polyester (PET), Polypropylen (PP), Polymilchsäure (PLA) und/oder Thermobindefasern, gebildet ist.

3. Vliesstoff nach Anspruch 1, **dadurch gekennzeichnet, dass** die nicht verfestigte Lage (11) aus Polymilchsäure (PLA) oder Polyester (PET) oder Polypropylen (PP) oder Polyethylen- oder Polyamidfasern oder mit Polyester- oder Polyolefinfasern hergestellt ist.

4. Vliesstoff nach Anspruch 1, **dadurch gekennzeichnet, dass** er über den superabsorbierenden Pulvern einen mit der der nicht verfestigten Lage (11) gegenüberliegenden Seite der verfestigten Lage (1) verbundenen Film aus Polyethylen oder Polymilchsäure umfasst.

5. Vliesstoff nach Anspruch 1, **dadurch gekennzeichnet, dass** die superabsorbierenden Pulver mit Polyethylen- oder Polymilchsäurepulvern im Verhältnis von 80% zu 20% gemischt sind.

6. Vliesstoff nach Anspruch 1, **dadurch gekennzeichnet, dass** es sich bei den superabsorbierenden Pulvern um gemeinhin als SAP bekannte handelt.

7. Vliesstoff nach Anspruch 1, **dadurch gekennzeichnet, dass** die verfestigte Lage (1) aus einer 3,2 dtex titrigen Viskosefaser mit einem Gewicht von 65 g/m² gebildet ist.

8. Vliesstoff nach Anspruch 1, **dadurch gekennzeichnet, dass** die Bindelinien (22) in einem Abstand von 2 bis 15 mm vorgesehen sind.

9. Vliesstoff nach Anspruch 1, **dadurch gekennzeichnet, dass** die Schicht von superabsorbierenden Pulvern über eine Dicke von ungefähr 5 bis 15 Mikrometern verfügt.

10. Vliesstoff nach Anspruch 4, **dadurch gekennzeichnet, dass** der Film aus Polyethylen oder Polymilchsäure über eine Dicke von 10 bis 25 Mikrometern verfügt.

11. Verfahren zur Herstellung eines mehrschichtigen Vliesstoffs variabler Dichte mit länglichen Kanälen, **gekennzeichnet durch** folgende Schritte:
- Bereitstellen einer kardierten Lage (6), gebildet von einem Material mit absorbierenden Eigenschaften und Fasern mit randomisierter Anordnung und verfestigt mit einem Spunlace-System;
- Bereitstellen einer verfestigten Lage (1), hergestellt aus einem Material mit nicht absorbierenden, aber auf jeden Fall hydrophilen Fasern;
- Ablegen der nicht verfestigten Lage (11) auf der verfestigten Lage (1) und Verbinden der nicht verfestigten Lage (11) mit der verfestigten Lage (1) **durch** Spunlace zur Verbindung der Lagen ausschließlich entlang von Bindelinien (22), dabei Bildung von Mulden in der nicht verfestigten Lage (11), während längliche Rippen (16) in den Lücken zwischen den Bindelinien (22) verbleiben, wobei die Fasern der nicht verfestigten Lage darin weich und lose bleiben, wobei ein Verbundprodukt (17) bereitgestellt wird;
- Ablegen einer Schicht einer Mischung von Pulvern von Polyethylen, Polymilchsäure, Polyvinylacetat, Polypropylen oder von Polyolefinpulvern, gemischt mit superabsorbierenden Pulvern, auf einer Rückseite der verfestigten Lage (1) des Verbundprodukts (17);
- Führen des Vliesstoffs **durch** ein Feld von Infrarotstrahlen, wobei die Polyethylen-, Polymilchsäure-, Polyvinylacetat-, Polypropylen- oder Polyolefinpulver geschmolzen werden und die superabsorbierenden Pulver an die Rückseite gebunden werden und ein Film gebildet wird.

12. Verfahren nach Anspruch 11, **dadurch gekennzeichnet, dass** zur Herstellung der kardierten Lage (6) Fasern aus einem Magazin (2) einem Kardiersystem (3, 4) zugeführt werden, welches die Fasern öffnet, die quer oder unregelmäßig angeordnet sind, wobei die Lage von Fasern (6) auf einem Förderband (5) liegend einseitig in einer Station (7) mit Hilfe von Strahlen unter hohem Druck stehenden Wassers verfestigt werden und einseitig eine verfestigte Lage (6bis) bilden, wobei die Lage einer zweiten Station (7bis) zugeführt wird und dort auf der anderen Seite verfestigt wird, wobei sich die verfestigte Lage (1) bildet, die auf einem Förderband (15) abgelegt wird.

13. Verfahren nach den Ansprüchen 11 und 12, **dadurch gekennzeichnet, dass** ein Magazin (9) Fasern für eine Kardiermaschine (10) bereitstellt, die die nicht verfestigte Lage von Fasern (11) ausbildet, die auf der verfestigten Lage (1) auf dem Förderband (15) abgelegt wird, wobei die übereinander geordneten Lagen (1-11) durch eine Station (12) von Hochdruckwasserstrahlen laufen, wobei ein fein perforierter Streifen (13) über entlang ungefähr 2 bis 15 mm voneinander beabstandeter paralleler Linien angeordnete kleine Löcher verfügt.

14. Verfahren nach den Ansprüchen 11 bis 13, **dadurch gekennzeichnet, dass** das Verbundprodukt (17) von einer Rolle (23) verkehrt, d.h. mit der Rückseite des Produkts (17) nach oben, auf ein Förderband (24) aufgelegt wird, auf dem das Produkt (17) unter einem Pulverzufuhrgerät (18) durchläuft, welches die Pulver auf der Rückseite des Produkts (17) abscheidet.

15. Verfahren nach den Ansprüchen 11 bis 14, **dadurch gekennzeichnet, dass** die superabsorbierenden Pulver durch einen mit einem Film aus Polyethylen oder Polymilchsäure oder anderen thermoplastischen Kunststoffen laminierten oder verbundenen Vliesstoff aufgebracht werden, wobei der Vliesstoff aus Viskose- oder Baumwollfasern hergestellt wird oder eine Mischung von Fasern mit thermoplastischen Fasern wie Polyester-, Polypropylen- oder anderen Kunststoff- oder Polyolefinfasern oder Polymilchsäurefasern darstellt, wobei der Vliesstoff auf der oberflächlichen Schicht von Klebstoff durch Heißschmelzen über Streichen, Sprühen oder Transfer mit einer Heißwalze, mit einer Gravurwalzentechnik aufgebracht wird, wobei die superabsorbierenden Pulver einheitlich verteilt und in länglichen Linien mit einer Breite von wenigen Millimetern angeordnet werden, wobei Leerstellen dazwischen gelassen werden, wobei der Vliesstoff in den durch die Abwesenheit der superabsorbierenden Pulver freigelassenen Stellen verbunden und fixiert wird, wobei die superabsorbierenden Pulver in den beiden Schichten belassen werden.

16. Verfahren nach den Ansprüchen 11 bis 15, **dadurch gekennzeichnet, dass** über eine zusätzliche Rolle (20) ein gegebenenfalls schon mit einer Lage Papier oder mit Vliesstoffen vorverbundener oder -laminierter Film aus Polyethylen oder Polymilchsäure bereitgestellt wird, der direkt mit der Rückseite des Produkts (17) verbunden wird, während das bzw. die schon mit den superabsorbierenden Pulvern abgeschiedene Polyethylen bzw. Polymilchsäure sich noch im schmelzflüssigen Zustand befindet.

## Revendications

1. Non-tissé, qui est un tissu de densité variable multicouche avec des canaux longitudinaux, comprend un pli non collé (11), ayant des caractéristiques hydrophiles non absorbantes, qui s'appuie sur un pli collé (1), ayant des caractéristiques absorbantes, lesdits plis étant collés les uns aux autres le long de lignes parallèles (22), **caractérisé en ce que** ledit pli collé (1) est constitué d'un pli cardé (6) collé avec un système de lacet par filage, ledit pli non collé (11) étant relié audit pli collé (1) le long desdites lignes de collage (22), et ayant des creux et des arêtes longitudinales (16) ; lesdites arêtes longitudinales (16) s'étendant le long d'espaces entre lesdites lignes de collage (22) ; ledit pli collé (1) ayant une surface arrière, opposée audit pli non collé (11), comprenant une couche de poudres superabsorbantes.

2. Non-tissé selon la revendication 1, **caractérisé en ce que** ledit pli collé (1) est constitué par des fibres de viscose ou de coton ou de cellulose ou par un mélange de ces fibres avec des fibres de poly(ester) ou de poly(oléfine) en général, telles que du poly(ester) (PET), du poly(propylène) (PP), du poly(acide lactique) (PLA) et/ou par des fibres thermocollantes.

3. Non-tissé selon la revendication 1, **caractérisé en ce que** le pli non collé (11) est constitué de fibres de poly(acide lactique) (PLA) ou de poly(ester) (PET), ou de poly(propylène) (PP), ou de poly(éthylène) ou de poly(amide) ou avec des fibres de poly(ester) ou de poly(oléfine).

4. Non-tissé selon la revendication 1, **caractérisé en ce qu'**il comprend un film de poly(éthylène) ou de poly(acide lactique), couplé à ladite surface dudit pli collé (1) opposé audit pli non collé (11), sur lesdites poudres superabsorbantes.

5. Non-tissé selon la revendication 1, **caractérisé en ce que** lesdites poudres superabsorbantes sont mélangées à 80 % avec 20 % de poudre de poly(éthylène) ou de poly(acide lactique).

6. Non-tissé selon la revendication 1, **caractérisé en ce que** lesdites poudres superabsorbantes sont des poudres couramment connues sous le nom de SAP.

7. Non-tissé selon la revendication 1, **caractérisé en ce que** ledit pli collé (1) est constitué d'une fibre de viscose à 3,2-dtex avec un grammage de 65 g/m².

8. Non-tissé selon la revendication 1, **caractérisé en ce que** les lignes de collage (22) sont disposées à une distance de 2 à 15 mm.

9. Non-tissé selon la revendication 1, **caractérisé en ce que** ladite couche de poudres superabsorbantes a une épaisseur d'approximativement 5 à 15 micromètres.

10. Non-tissé selon la revendication 4, **caractérisé en ce que** ledit film de poly(éthylène) ou de poly(acide lactique) a une épaisseur de 10 à 25 micromètres.

11. Procédé de fabrication d'un tissu de densité variable multicouche non-tissé avec des canaux longitudinaux, **caractérisé en ce qu'**il comprend les étapes consistant à :
- fournir un pli cardé (6) constitué d'un matériau ayant des caractéristiques absorbantes et de fibres ayant une orientation aléatoire, et collées avec un système de lacet par filage ;
- fournir un pli collé (1) constitué d'un matériau avec des fibres non absorbantes mais dans tous les cas hydrophiles ;
- déposer ledit pli non collé (11) sur ledit pli collé (1) et relier ledit pli non collé (11) audit pli collé (1) par lacet par filage pour coller lesdits plis seulement le long des lignes de collage (22), en y formant des creux dans ledit pli non collé (11), tandis que des arêtes longitudinales (16) restent dans les espaces entre lesdites lignes de collage (22), les fibres du pli non collé restant souples et lâches à l'intérieur, fournissant un produit composite (17) ;
- déposer une couche d'un mélange de poudres de poly(éthylène), de poly(acide lactique), de poly(acétate de vinyle), de poly(propylène) ou de poudres de poly(oléfine), mélangées avec des poudres superabsorbantes, sur une surface arrière dudit pli collé (1) dudit produit composite (17);
- faire passer ledit non-tissé à travers un champ de rayons infrarouge, faire fondre lesdites poudres de poly(éthylène), de poly(acide lactique), de poly(acétate de vinyle), de poly(propylène) ou de poly(oléfine) et coller lesdites poudres superabsorbantes à ladite surface arrière, formant un film.

12. Procédé selon la revendication 11, **caractérisé en ce que** ledit pli cardé (6) est produit avec des fibres distribuées par un magasin (2) sur un système de cardage (3, 4), qui ouvre les fibres, qui sont agencées transversalement ou de manière aléatoire ; ledit pli de fibres (6) s'appuyant sur un convoyeur (5) et étant collé sur une face dans une station (7) au moyen de jets d'eau haute pression, formant un pli collé (6 bis) sur une face ; ledit pli étant envoyé vers une seconde station (7 bis), qui le colle sur l'autre face, formant le pli collé (1), qui s'appuie sur un convoyeur (15).

13. Procédé selon les revendications 11 et 12, **caractérisé en ce qu'**un magasin (9) fournit des fibres à une machine de cardage (10), qui forme le pli non collé de fibres (11), qui est disposé sur le pli collé (1) sur la courroie de convoyeur (15) ; lesdits plis superposés (1 et 11) traversant une station (12) de jets d'eau haute pression, où une bande finement perforée (13) comporte des petits trous agencés le long de lignes parallèles qui sont espacées d'approximativement 2 à 15 mm.

14. Procédé selon les revendications 11 à 13, **caractérisé en ce que** ledit produit composite (17) est fourni à partir d'un dévidoir (23) à l'envers, c'est-à-dire de sorte que la face arrière du produit (17) est agencée au-dessus et appuyée sur une courroie de convoyeur (24), qui fait passer le produit (17) sous un dispositif d'alimentation en poudre (18), qui dépose lesdites poudres sur ladite surface arrière du produit (17).

15. Procédé selon les revendications 11 à 14, **caractérisé en ce que** lesdites poudres superabsorbantes sont appliquées à travers un non-tissé qui est stratifié ou couplé à un film de poly(éthylène) ou de poly(acide lactique) ou d'autres polymères thermoplastiques, ledit non-tissé étant constitué de fibres de viscose ou de coton, ou étant un mélange de fibres avec des fibres thermoplastiques telles que des fibres de poly(ester), de poly(propylène) ou d'autres fibres polymères ou de poly(oléfine), ou des fibres de poly(acide lactique) ; ledit non-tissé étant appliqué à la couche de surface de l'adhésif par fusion à chaud par étalement, vaporisation ou transfert avec un cylindre chaud, avec une technique au rouleau de grainage ; lesdites poudres superabsorbantes étant distribuées de manière uniforme et agencées en lignes longitudinales ayant une largeur de quelques millimètres, avec des espaces vides intercalés, ledit tissu étant couplé et fixé dans les espaces vides laissés par l'absence des poudres superabsorbantes, laissant les poudres superabsorbantes à l'intérieur des deux couches.

16. Procédé selon les revendications 11 à 15, **caractérisé en ce qu'**un dévidoir supplémentaire (20) fournit un film de poly(éthylène) ou de poly(acide lactique), éventuellement déjà couplé ou stratifié au préalable avec un pli de papier, ou avec des non-tissés, qui est couplé directement à la surface arrière du produit (17), pendant que le poly(éthylène) ou le poly(acide lactique), déjà déposé avec les poudres superabsorbantes, est encore fondu.
